# EUROPÄISCHE PATENTSCHRIFT

(11) **EP 1 287 791 B1**
(45) Veröffentlichungstag und Bekanntmachung des Hinweises auf die Patenterteilung: **09.09.2009**
(21) Anmeldenummer: 01810823.3
(22) Anmeldetag: 23.08.2001
(51) Int. Cl.: A61F 2/16

(54) **Vorrichtung zur Einführung einer Linse in ein Auge**
Device for implanting a lens into an eye
Dispositif pour implanter une lentille dans l'oeil

(43) Veröffentlichungstag der Anmeldung: 05.03.2003
(73) Patentinhaber: Anton Meyer & Co. AG, 2562 Port (CH); Asico LLC, Westmont, IL 60559 (US)
(72) Erfinder: Meyer, Rudolf, 2562 Port (CH)
(74) Vertreter: Clerc, Natalia

(56) Entgegenhaltungen:
- EP-A- 0 477 466
- WO-A-99/59668
- US-A- 5 643 276
- US-A- 6 059 791
- US-B1- 6 203 549

## Beschreibung

### Technisches Gebiet

Die Erfindung betrifft eine Vorrichtung zur Einführung einer Linse in ein Auge gemäss Oberbegriff des Patentanspruches 1.

### Stand der Technik

In der Augenchirurgie werden heute trübe natürliche Augenlinsen durch künstliche Linsen ersetzt. Bei diesem Eingriff wird zuerst die trübe Linse des Patienten entfernt. Anschliessend setzt der Chirurg die künstliche Linse mit Hilfe zweier Pinzetten in das Auge ein. Dabei dient die erste Pinzette zum Falten der Linse und die zweite Pinzette zum Einführen der Linse. Dies erfordert vom Chirurgen eine überaus sichere Hand und eine grosse Übung bei der Führung der zwei Pinzetten.

Eine gewisse Führung beim Einsetzen von künstlichen Linsen ermöglicht ein sogenannter Injektor. Diese Vorrichtung zur Einführung einer Linse in ein Auge besteht im wesentlichen aus einem Griffkörper und einem im Griffkörper über ein Gewinde verschiebbaren Kolben. Im vorderen Bereich des Griffkörpers ist eine Linsenaufnahme vorhanden, in welche eine einzuführende Linse eingelegt wird. Durch Drehen des Kolbens lässt sich nun diese Linse durch eine vordere Öffnung der Linsenaufnahme durchschieben, wobei die Linse dabei gefaltet wird. Durch weiteres Vorschieben wird die Linse im gefalteten Zustand ins Auge eingebracht. Das Gewinde ermöglicht dabei zwar eine genaue Führung des Kolbens. Nachteilig ist jedoch, dass der Chirurg zum Drehen des Kolbens beide Hände benötigt. Zudem ist es relativ schwierig, den Injektor während des Drehens gerade zu halten.

Eine Vorrichtung gemäß des Oberbegriffs des Anspruchs 1 ist in dem Dokument WO-A-99/59668 offenbart.

### Darstellung der Erfindung

Es ist deshalb Aufgabe der Erfindung, eine Vorrichtung zur Einführung einer Linse in eine Auge der eingangs erwähnten Art zu schaffen, welche einhändig bedienbar ist und trotzdem eine genaue Führung des Kolbens ermöglicht.

Diese Aufgabe wird durch die erfindungsgemäße Vorrichtung mit den Merkmalen des Patentanspruches 1 gelöst.

Erfindungsgemäss ist der Kolben in einer Kugellagerbüchse in Längsrichtung verschiebbar gelagert. Dadurch ist der Injektor einhändig bedienbar. Um den Kolben zu verschieben muss, wie bei einer Spritze, nur auf den Kolben gedrückt werden. Durch die Lagerung in der Kugellagerbüchse weist der Kolben verbesserte Gleiteigenschaften auf, was wiederum ein gleichmässiges Vorschieben des Kolbens und somit ein gleichmässiges Einführen der Linse erlaubt.

In einer bevorzugten Ausführungsform ist eine präzise Führung des Kolbens durch eine im Kolben eingebrachte Führungsnut gewährleistet, in welche ein im Griffkörper angeordnetes Führungselement eingreift.

In einer ersten Ausführungsform ist die Führungsnut geradlinig ausgebildet, so dass der Kolben geradlinig geführt verschiebbar ist.

In einer zweiten Ausführungsform weist die Führungsnut einen Wendel auf, so dass sich der Kolben zudem beim Verschieben geführt drehen lässt. Ist der Wendel im hinteren Bereich des Kolbens angeordnet, so ermöglicht diese Drehung am Schluss der Einführbewegung, die Linse im Auge zu entfalten.

Weitere vorteilhafte Ausführungsformen gehen aus den abhängigen Patentansprüchen hervor.

### Kurze Beschreibung der Zeichnung

Im folgenden wird der Erfindungsgegenstand anhand von bevorzugten Ausführungsbeispielen, welche in der beiliegenden Zeichnung dargestellt sind, erläutert. Es zeigen:
- Figur 1: einen erfindungsgemässen Injektor gemäss einer ersten Ausführungsform;
- Figur 2: einen Ausschnitt gemäss Figur 1 in vergrösserter Darstellung;
- Figur 3: einen erfindungsgemässen Injektor gemäss einer zweiten Ausführungsform und
- Figur 4: einen Ausschnitt gemäss Figur 3 in vergrösserter Darstellung.

### Wege zur Ausführung der Erfindung

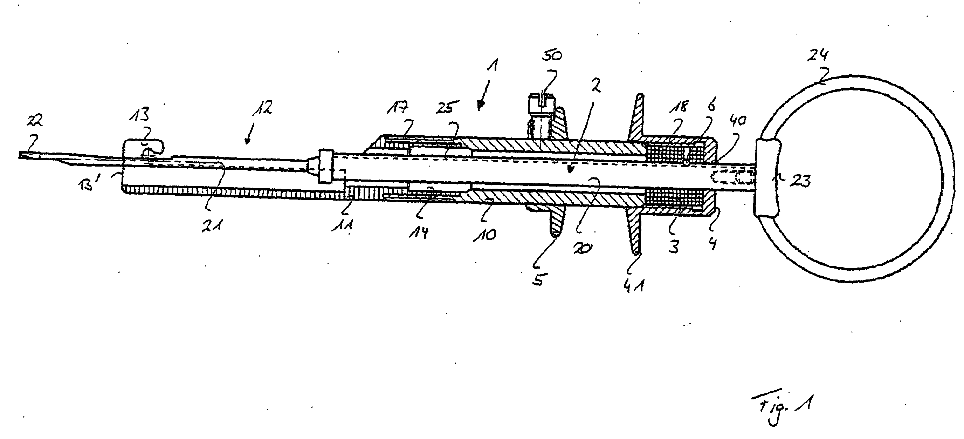

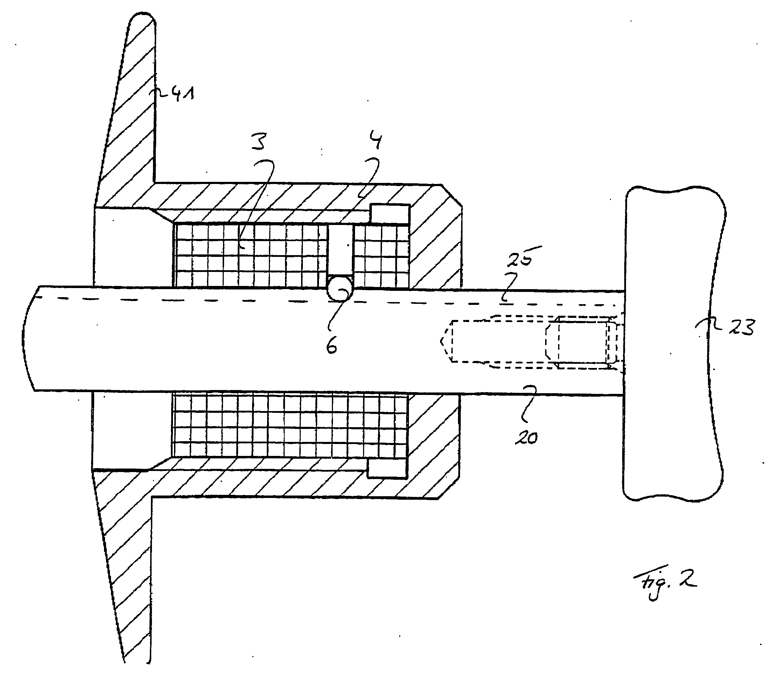

In den Figuren 1 und 2 ist ein erfindungsgemässer Injektor gemäss einer ersten Ausführungsform dargestellt. Er umfasst einen Griffkörper 1, in welchem ein Kolben 2 verschiebbar gelagert ist. Sowohl Griffkörper 1 wie auch Kolben 2 sind vorzugsweise aus einem Metall, insbesondere Titan, gefertigt.

Der Griffkörper 1 weist eine Hülse 10 auf, welche an ihrem hinteren Ende durch eine Abschlusskappe 4 geschlossen ist. An ihrem vorderen Ende geht die Hülse 10 in ein Griffvorderteil 11 über. Im hier dargestellten Beispiel ist der Griffvorderteil 11 ein separates Bauteil, welches über ein erstes Gewinde 17 mit der Hülse 10 verschraubt ist.

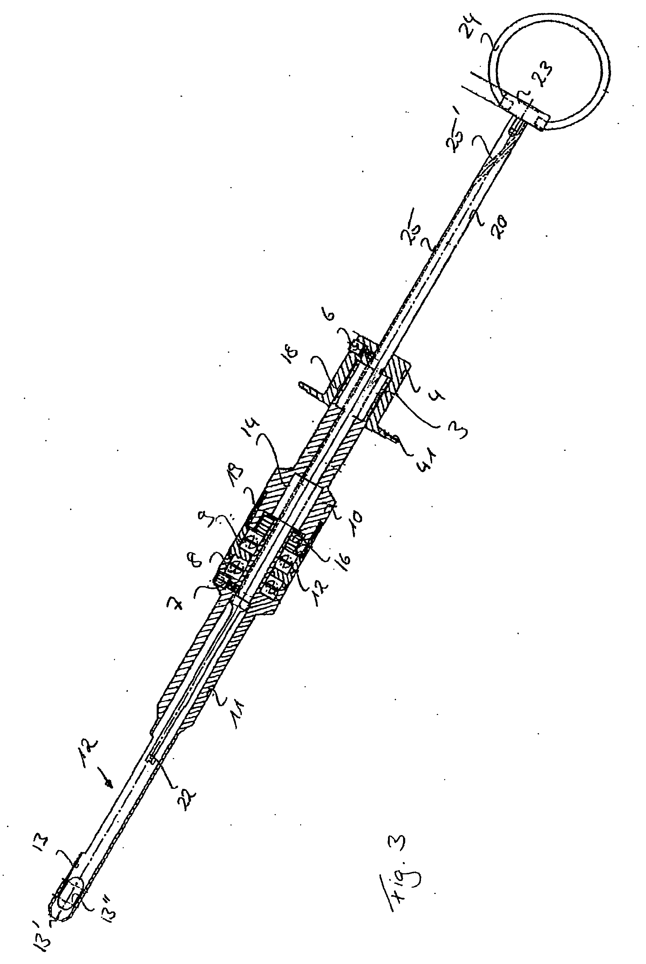

Das Griffvorderteil 11 weist an seinem vorderen, der Hülse 10 abgewandten Ende eine Linsenaufnahme 13 zur Aufnahme einer künstlichen Linse auf. Hinter der Linsenaufnahme 13 weist das Griffvorderteil 11 ein langgezogenes, offenes Einführfenster 12 auf, über welches die Linse in die Linsenaufnahme 13 eingelegt wird. Die Linsenaufnahme 13 weist an ihrem vorderen Ende eine Durchlassöffnung 13' auf, durch welche die Linse hinausgestossen wird. Wie in Figur 3 ersichtlich ist, lässt sich in der Linsenaufnahme 13 ein Fenster 13'' einbringen, um die Lage der eingelegten Linse zu überprüfen.

Die Hülse 10 weist vorzugsweise einen geschlossenen Mantel auf. Im hinteren Bereich der Hülse 10 ist eine Kugellagerbüchse 3 angeordnet. Diese Kugellagerbüchse 3 ist von der Abschlusskappe 4 umschlossen, welche mit der Hülse 10 über ein zweites Gewinde 18 verschraubt ist. Die Kugellagerbüchse 3 ist in diesem Ausführungsbeispiel bezüglich der Hülse 10 lagefixiert. Dies lässt sich beispielsweise dadurch erreichen, dass die Abschlusskappe 4 im Gegensatz zur Kugellagerbüchse 3 nicht einen runden Querschnitt aufweist. In einer anderen Variante sind am äusseren Mantel der Kugellagerbüchse 3 Noppen als Verdrehsicherung angeordnet.

Die Kugellagerbüchse 3 weist einen definierten Aussendurchmesser auf. Vorzugsweise besitzt sie hierfür in ihren Mantel eingegossene, der Mantelfläche vorstehende und in Längsrichtung verlaufende Metallstege. Dadurch lässt sich die Kugellagerbüchse 3 auch aus Kunststoff fertigen. Die Kugellagerbüchse 3 weist vorzugsweise mehrere, in Längsrichtung verlaufende Kugellager auf. Bewährt haben sich vier, über den Innenumfang gleichmässig verteilt angeordnete Kugellagerbahnen, welche sich mindestens annähernd über die gesamte Länge der Kugellagerbüchse 3 erstrecken.

Die Abschlusskappe 4 weist an ihrem vorderen Ende einen vorstehenden, umlaufenden Flansch 41 auf, welcher wie bei einer Spritze als Fingeranschlag für die Finger des Chirurgen, vorzugsweise den Zeige- und Mittelfinger, dient. Am anderen Ende ist die Abschlusskappe 4 bis auf eine Durchführöffnung 40 geschlossen. Vorzugsweise ist ein zweiter Flansch 5 an der Hülse 10 angeordnet oder angeformt, welcher als vorderer Anschlag für die Finger des Chirurgen dient. Ist der zweite Flansch 5, wie hier dargestellt, ein separates Element, welches über eine Befestigungsschraube 50 lösbar an der Hülse 10 befestigbar ist und entlang dieser verschiebbar ist, so lässt sich der Abstand zwischen dem ersten und zweiten Flansch der Fingerdicke des Chirurgen anpassen.

Der Kolben 2 durchsetzt die Hülse 10 und ragt mit seinem hinteren Ende aus der Durchführöffnung der Abschlusskappe 4 heraus. Der Kolben 2 besteht im wesentlichen aus einem hinteren Kolbenkopf 23, einem Kolbenmittelteil 20, einer Kolbennadel 21 und einer Kolbenspitze 22. Die Kolbennadel 21 ist vorzugsweise lösbar mit dem Kolbenmittelteil 20 verbunden, so dass je nach Art der Linse eine neue Nadel verwendet werden kann. Zudem lässt sie sich auch als Einwegnadel einsetzen. An der Spitze der Nadel 21 ist die Kolbenspitze 22 angebracht oder einstückig angeformt. Auch ihre Form variiert in Abhängigkeit von der Art der Linse.
Der Kolbenkopf 23 bildet das hintere Ende des Kolbens 2. Er ist mit einem Fingerring 24 verbunden. Der Kolbenkopf 23 dient dem Chirurgen als Druckfläche für den Daumen, um den Kolben 2 einhändig in Längsrichtung in der Hülse 10 nach vorne zu stossen. Der Fingerring 24 ermöglicht eine Rückholung des Kolbens 2 mit dem Daumen, ohne dass die Lage der den Injektor haltenden Finger verändert werden muss. Wird der Kolben 2 nach vorne gestossen, so wird die in der Linsenaufnahme 13 liegende künstliche Linse gefaltet, nach vorne aus dem Injektor rausgeschoben und mit genau dosierbarem Druck und in geführter Richtung in das Auge eingesetzt. In Figur 1 ist der Injektor nach Einführung einer Linse mit vorgeschobenem Kolben 2 dargestellt.

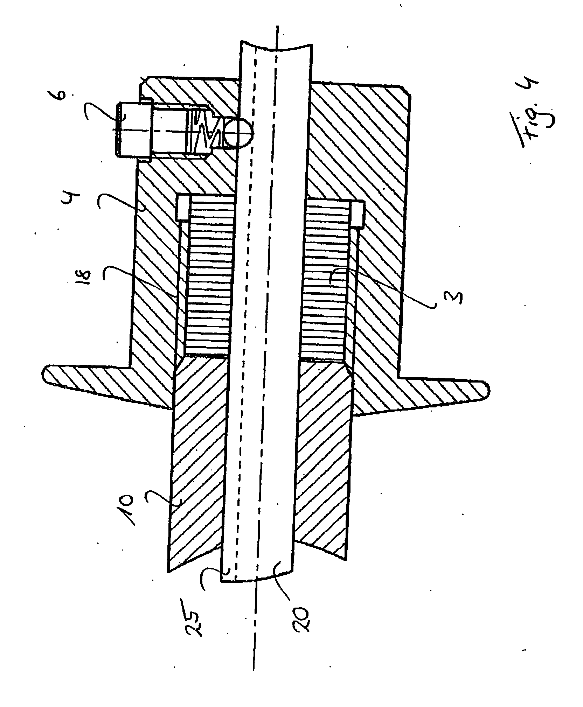

Die Kugellager der Kugellagerbüchse 3 ermöglichen eine reibungsarme Verschiebung des Kolbens. Als Führungsmittel ist am Kolben 2 eine geradlinig verlaufende Führungsnut 25 vorhanden, welche sich mindestens über die gesamte Verschiebungsstrecke des Kolbens 2 erstreckt. Im hier dargestellten Beispiel erstreckt sie sich über die Länge des Kolbenmittelteils 20. In der Kugellagerbüchse 3 ist ein Führungselement 6, hier in Form einer vorstehenden Metallkugel angeordnet, welches druckbeaufschlagt in die Führungsnut 25 eingreift und so eine Verdrehung des Kolbens 2 während des Vorschiebens verhindert. Das Führungselement 6 lässt sich auch ausserhalb der Kugellagerbüchse 3 anordnen, wobei es mindestens in diesem Fall federbeaufschlagt ist. Ein derartiges Führungselement 6 ist in den Figuren 3 und 4 dargestellt.
Um einen Staudruck und somit eine hemmende Gegenkraft auf den Kolben 2 zu verhindern, weist die Hülse 10 ferner mindestens eine Entlastungskammer 14 auf, um die beim Vorschieben des Kolben 2 zusammengepresste Luft zu entspannen.

Die Figuren 3 und 4 zeigen den erfindungsgemässen Injektor in einer zweiten Ausführungsform. Gleiche Teile sind mit gleichen Bezugsziffern wie in den Figuren 1 und 2 versehen, so dass auf diese nicht mehr näher eingegangen wird. Im Gegensatz zur ersten Ausführungsform weist der Kolben 2 eine Führungsnut 25 auf, welche am hinteren Ende mit einem Wendel 25' versehen ist. Das Führungselement 6 greift wiederum in die Führungsnut 25 ein. Ist nun der Kolben 2 so weit vorgeschoben, dass die Nut in den Wendel 25' übergeht, so wird der Kolben 2 geführt gedreht. Um die Lagerung weiterhin zu gewährleisten, ist in dieser Ausführungsform die Kugellagerbüchse 3 nicht lagefixiert, sondern drehbar an der Hülse 10 angeordnet, so dass sie mit dem Kolben 2 mitdreht.

In der hier dargestellten Ausführungsform dreht das Griffvorderteil 11 mit dem Kolben 2 mit. Hierfür ist zwischen Griffvorderteil 11 und Hülse 10 ein Griffmittelteil 15 vorhanden, welches über ein drittes Gewinde 18 mit der Hülse 10 verbunden ist. Zwischen dem Griffvorderteil 11 und dem Griffmittelteil 15 ist ein erstes Axiallager 8 und zwischen dem Griffmittelteil 15 und Hülse 10 ein zweites Axiallager 9 angeordnet. Das zweite Axiallager 9 ist dabei mittels einer Mutter 16 in seiner Lage gehalten.

Im Griffvorderteil 11 ist ein Mitnehmer 7 angeordnet. Es ist identisch aufgebaut wie das gefederte Führungselement 6 und greift ebenfalls in die Führungsnut 25 des Kolbens 2 ein. Wird nun der Kolben 2 durch den Wendel 25' gedreht, so wird auch das Griffvorderteil 11 durch die Verbindung über den Mitnehmer 7 mitgedreht. Diese Drehung am Schluss des Einführens der Linse vereinfacht die Entfaltung der Linse und ihre Plazierung im Auge.

Soll lediglich der Kolben 2, nicht aber der Griffvorderteil 11 gedreht werden, so ist es auch möglich, einen zweiten Wendel am Kolben anzubringen. Dabei müssen die zwei Wendel denselben Abstand aufweisen wie das Führungselement 6 zum Mitnehmer 7. Zudem ist die Länge des Kolbens 2 so zu bemessen, dass der zweite Wendel bei ausgezogener Grundstellung des Kolbens 2, das heisst vor Beginn des Vorschiebens, vor dem ersten Führungselement 6 zu liegen kommt.

Die erfindungsgemässe Vorrichtung ermöglicht eine einhändige Bedienung, wobei die Vorrichtung zudem gute Gleiteigenschaften aufweist und eine stets in Längsrichtung geführte oder gedrehte Bewegung des Kolbens ermöglicht.

### Bezugszeichenliste

- 1: Griffkörper
- 10: Hülse
- 11: Griffvorderteil
- 12: Einführfenster
- 13: Linsenaufnahme
- 13': Durchlassöffnung
- 13'': Fenster
- 14: Entlastungskammer
- 15: Griffmittelteil
- 16: Mutter
- 17: Erstes Gewinde
- 18: Zweites Gewinde
- 19: Drittes Gewinde
- 2: Kolben
- 20: Kolbenmittelteil
- 21: Kolbennadel
- 22: Kolbenspitze
- 23: Kolbenkopf
- 24: Fingerring
- 25: Führungsnut
- 25': Wendel
- 3: Kugellagerbüchse
- 4: Abschlusskappe
- 40: Durchführöffnung
- 41: Erster Flansch
- 5: Zweiter Flansch
- 50: Befestigungsschraube
- 6: Führungselement
- 7: Mitnehmer
- 8: Erstes Axiallager
- 9: Zweites Axiallager

## Patentansprüche

1. Vorrichtung zur Einführung einer Linse in ein Auge, wobei die Vorrichtung einen Griffkörper (1) mit einer Linsenaufnahme (13) zur Aufnahme der Linse und einen im Griffkörper (1) verschiebbaren Kolben (2) zur geführten Einführung der Linse in das Auge aufweist, wobei der Kolben einen Kolbenkopf (23) aufweist, welcher einem Chirurgen als Druckfläche für den Daumen dient, um den Kolben (2) im Griffkörper (1) nach vorne zu stossen, **dadurch gekennzeichnet, dass** im Griffkörper (1) eine Kugellagerbüchse (3) angeordnet ist und dass der Kolben (2) in dieser Kugellagerbüchse (3) in Längsrichtung verschiebbar gelagert ist, und dass die Kugellagerbüchse (3) im hinteren, der Linsenaufnahme (13) abgewandten Ende des Griffkörpers (1) angeordnet ist.

2. Vorrichtung nach Anspruch 1, **dadurch gekennzeichnet, dass** der Kolben (2) eine Führungsnut (25) aufweist, in welche ein im Griffkörper (1) angeordnetes Führungselement (6) eingreift.

3. Vorrichtung nach Anspruch 2, **dadurch gekennzeichnet, dass** das Führungselement (6) in der Kugellagerbüchse (3) angeordnet ist.

4. Vorrichtung nach Anspruch 1, **dadurch gekennzeichnet, dass** die Kugellagerbüchse (3) lagefixiert im Griffkörper (1) angeordnet ist.

5. Vorrichtung nach Anspruch 2, **dadurch gekennzeichnet, dass** die Führungsnut (25) geradlinig verläuft.

6. Vorrichtung nach Anspruch 2, **dadurch gekennzeichnet, dass** die Führungsnut (25) mindestens im hinteren Bereich des Kolbens (2) einen Wendel (25') aufweist, so dass der Kolben (2) geführt drehbar ist.

7. Vorrichtung nach Anspruch 6, **dadurch gekennzeichnet, dass** der Wendel (25') im hinteren Bereich des Kolbens (2) angeordnet ist.

8. Vorrichtung nach Anspruch 6, **dadurch gekennzeichnet, dass** der Griffkörper (1) eine Hülse (10) und eine über Axiallager (8,9) mit der Hülse (10) verbundenen Griffvorderteil (11) aufweist und dass im Griffvorderteil (11) ein Mitnehmer (7) angeordnet ist, welcher in die Führungsnut (25) eingreift, so dass der Griffvorderteil (11) mit dem Kolben (2) geführt drehbar ist.

9. Vorrichtung nach Anspruch 6, **dadurch gekennzeichnet, dass** die Kugellagerbüchse (3) drehbar im Griffkörper (1) angeordnet ist und mit dem Kolben (2) gemeinsam geführt drehbar ist.

## Claims

1. A device for inserting a lens into an eye, said device comprising a grip body (1) with a lens receiving portion (13) for receiving the lens, and a plunger (2) which is displaceable in the grip body (1) for guided insertion of the lens into the eye, the plunger comprising a plunger head (23) serving as a pressing surface for the thumb of a surgeon to press the plunger (2) forward in the grip body (1), **characterized in that** a ball-bearing bush (3) is arranged in the grip body (1), that the plunger (2) is mounted so as to be longitudinally displaceable in said ball-bearing bush (3), and that the ball-bearing bush (3) is arranged in the rear end of the grip body (1) directed away from the lens receiving portion (13).

2. The device as claimed in claim 1, **characterized in that** the plunger (2) has a guide groove (25) into which there engages a guide element (6) arranged in the grip body (1).

3. The device as claimed in claim 2, **characterized in that** the guide element (6) is arranged in the ball-bearing bush (3).

4. The device as claimed in claim 1, **characterized in that** the ball-bearing bush (3) is arranged in a fixed position in the grip body (1).

5. The device as claimed in claim 2, **characterized in that** the guide groove (25) is rectilinear.

6. The device as claimed in claim 2, **characterized in that** the guide groove (25) has a spiral (25') at least in the rear area of the plunger (2), so that the plunger (2) can be rotated in a guided manner.

7. The device as claimed in claim 6, **characterized in that** the spiral (25') is arranged in the rear area of the plunger (2).

8. The device as claimed in claim 6, **characterized in that** the grip body (1) comprises a sleeve (10) and a grip front part (11) connected to the sleeve (10) via axial bearings (8, 9), and that a carrier (7) is arranged in the grip front part (11) which engages in the guide groove (25) so that the grip front part (11) can be rotated in a guided manner with the plunger (2).

9. The device as claimed in claim 6, **characterized in that** the ball-bearing bush (3) is arranged rotatably in the grip body (1) and can be rotated in a guided manner together with the plunger (2).

## Revendications

1. Dispositif pour introduire une lentille dans un oeil, le dispositif présentant un corps de poignée (1) avec une réception de lentille (13) pour recevoir la lentille et un piston (2) mobile dans le corps de poignée (1) pour l'introduction guidée de la lentille dans l'oeil, le piston présentant une tête de piston (23), laquelle sert de surface de pression pour le pouce d'un chirurgien pour pousser le piston (2) vers l'avant dans le corps de poignée (1), **caractérisé en ce qu'**une douille de roulement à bille (3) est placée dans le corps de poignée (1) et que le piston (2) est placé dans cette douille de roulement à bille (3) de manière mobile dans la direction longitudinale, et que la douille de roulement à bille (3) est placée dans l'extrémité arrière du corps de poignée (1) opposée à la réception de lentille (13).

2. Dispositif selon la revendication 1, **caractérisé en ce que** le piston (2) présente une rainure de guidage (25) dans laquelle un élément de guidage (6) disposé dans le corps de poignée (1) se met en prise.

3. Dispositif selon la revendication 2, **caractérisé en ce que** l'élément de guidage (6) est disposé dans la douille de roulement à bille (3).

4. Dispositif selon la revendication 1, **caractérisé en ce que** la douille de roulement à bille (3) est placée fixée en position dans le corps de poignée (1).

5. Dispositif selon la revendication 2, **caractérisé en ce que** la rainure de guidage (25) est en ligne droite.

6. Dispositif selon la revendication 2, **caractérisé en ce que** la rainure de guidage (25) présente au moins dans la partie arrière du piston (2) une hélice (25') de façon à ce que le piston (2) puisse être guidé en rotation.

7. Dispositif selon la revendication 6, **caractérisé en ce que** l'hélice (25') est disposée dans la partie arrière du piston (2).

8. Dispositif selon la revendication 6, **caractérisé en ce que** le corps de poignée (1) présente un manchon (10) et une partie avant de poignée (11) reliée au manchon (10) par des roulements axiaux (8, 9) et qu'un entraîneur (7) est placé dans la partie avant de poignée (11), lequel se met en prise dans la rainure de guidage (25), de façon à ce que la partie avant de poignée (11) soit guidée de manière rotative avec le piston (2).

9. Dispositif selon la revendication 6, **caractérisé en ce que** la douille de roulement à bille (3) est placée de manière rotative dans le corps de poignée (1) et peut être guidée de manière rotative conjointement avec le piston (2).
